# EUROPEAN PATENT SPECIFICATION

(11) **EP 3 920 859 B1**
(45) Date of publication and mention of the grant of the patent: **03.09.2025**
(21) Application number: 20751899.4
(22) Date of filing: 06.02.2020
(51) Int. Cl.: A61F 9/007, A61B 3/10, A61B 34/30

(54) **EXTERNAL AND INTERNAL INTRAOPERATIVE OPTICAL COHERENCE TOMOGRAPHY IMAGING FOR SUBRETINAL MATERIAL DELIVERY**
EXTERNE UND INTERNE INTRAOPERATIVE OPTISCHE KOHÄRENZTOMOGRAFIE FÜR SUBRETINALE MATERIALABGABE
IMAGERIE DE TOMOGRAPHIE PAR COHÉRENCE OPTIQUE PEROPÉRATOIRE EXTERNE ET INTERNE POUR DISTRIBUTION DE MATÉRIAU SOUS-RÉTINIEN

(30) Priority: 06.02.2019 US 201962801894 P
(43) Date of publication of application: 15.12.2021
(73) Proprietor: The Johns Hopkins University, Baltimore, Maryland 21218 (US)
(72) Inventor: KANG, Jin Ung, Baltimore, Maryland 21218 (US); SINGH, Mandeep, Baltimore, Maryland 21218 (US)
(74) Representative: Murgitroyd & Company
(86) International application number: PCT/US2020/017001
(87) International publication number: WO 2020/163587

(56) References cited:
- WO-A1-2014/043517
- US-A1- 2010 056 900
- US-A1- 2011 106 102
- US-A1- 2011 106 102
- US-A1- 2012 130 258
- US-A1- 2012 143 084
- US-A1- 2013 123 759
- US-A1- 2013 123 759
- US-A1- 2013 148 106
- US-A1- 2016 074 212
- US-A1- 2017 258 988
- TANG, Q. ET AL.: "Real-time epidural anesthesia guidance using optical coherence tomography needle probe", QUANTITATIVE IMAGING IN MEDICINE AND SURGERY, vol. 5, no. 1, 2015, pages 118 - 124, XP055731219, Retrieved from the Internet <URL:https://www.ncbi.nlm.nih.gov/pmc/articles/PMC4312299/pdf/qims-05-01-118.pdf> DOI: 10.3978/j.issn.2223-4292.2014.11.28

## Description

### BACKGROUND

Many degenerative retinal diseases exist for which no complete treatment is available. Ocular gene therapy and cell therapy has emerged as a promising method for treating hereditary retinal diseases. In ocular gene and cell therapy, gene vectors and cells can be delivered to target areas in the eye by microinjection, including subretinal injection. During a typical subretinal injection, a surgeon is totally dependent on manual manipulation of the surgical tools and visual feedback through, for example, a stereomicroscope. Such approaches, however, can be limited by physiological hand tremor of the surgeon and the limited ability of humans to visually resolve a micrometer scale on the axial axis. A variety of microinjection systems have been proposed. Such systems, however, have not achieved the precise depth control required for subretinal injections.

Further, current methods for delivering therapeutic agents to the subretinal space for treating degenerative retinal diseases rely on accessing the subretinal space via a trans-retinal approach. The trans-retinal approach requires making a surgical incision in the retina to gain access to the subretinal space. This retinal incision is associated with a high rate of complications, including bleeding, scarring, retinal detachment, and off target delivery of the agent due to reflux into other ocular compartments. Subretinal injection through the sclera may provide a safer alternative to injections through the intraocular space. Performing a transscleral subretinal injection, i.e., across the sclera, or white, of the eye, however, is highly challenging since the sclera is visually opaque, highly scattering, and prevents normal microscope and optical coherence tomography (OCT) views of the target area.

US Patent Application US 2011/106102 A1 (The Johns Hopkins University) describes a microsurgical tool incorporating common path optical tomography capabilities. International Patent Application WO 2014/043517 A1 (The Johns Hopkins University) describes a motion-compensated optical coherence tomography system. US Patent Application US 2012/0143084 A1 describes a hand held robotic system to control the position of a surgical tool to correct deviations from a predetermined pose or path, or comes too close to a hazardous area.

### SUMMARY

The presently disclosed subject matter provides a common-path swept source optical coherence tomography (CP-SSOCT) distal sensor-guided injection device as defined in claims 1, and optional dependent claims. Methods disclosed in the description have been included for better understanding the invention and are not claimed.

In some aspects, the presently disclosed subject matter provides a common-path swept source optical coherence tomography (CP-SSOCT) distal sensor guided manual injection system for transscleral subretinal injection. The presently disclosed system enables precise subretinal injection with micrometer-level injection depth control. Such depth control can be achieved by using a high-resolution CP-SSOCT distal sensor coupled to a precise translational stage and using signal processing from a graphics-processing unit (GPU). The presently disclosed CP-SSOCT guided microinjector is capable of precisely guiding and locking in the injection needle tip to the desired target depth within the subretinal space during injection.

Accordingly, in some aspects, the presently disclosed subject matter provides a common-path swept source optical coherence tomography (CP-SSOCT) distal sensor-guided injection device comprising:
an optical fiber comprising a distal end, wherein the distal end of the optical fiber is configured to be proximate to or in contact with a selected portion of tissue, and wherein the optical fiber is arranged to direct light to the selected portion of tissue and to detect light reflected from the selected portion of tissue to provide information regarding a relative distance of the distal end of the optical fiber to the selected portion of tissue;
an injection needle configured to deliver one or more therapeutic agents to the selected portion of the tissue, wherein the injection needle is operably coupled and substantially parallel to the optical fiber such that the relative distance of the distal end of the optical fiber to the selected portion of tissue as determined by a CP-SSOCT system provides a position of a tip of the injection needle relative to the selected portion of tissue, wherein the tip comprises a distal end configured for insertion into the selected portion of tissue;
a translational stage configured to axially position the optical fiber and injection needle at a relative distance to the selected portion of tissue, wherein the optical fiber and injection needle are operably coupled to the translational stage; and
an articulated arm, wherein the translational stage is operably coupled to the articulated arm.

Certain aspects of the presently disclosed subject matter having been stated hereinabove, which are addressed in whole or in part by the presently disclosed subject matter, other aspects will become evident as the description proceeds when taken in connection with the accompanying Examples and Figures as best described herein below.

### BRIEF DESCRIPTION OF THE FIGURES

The patent or application file contains at least one drawing executed in color. Copies of this patent or patent application publication with color drawings will be provided by the Office upon request and payment of the necessary fee.

Having thus described the presently disclosed subject matter in general terms, reference will now be made to the accompanying Figures, which are not necessarily drawn to scale, and wherein:
FIG. 1A illustrates a partially sectional view of a plane of delivery according to an embodiment of the presently disclosed subject matter;
FIG. 1B illustrates a partially sectional view of an entry point and a target point according to an embodiment of the presently disclosed subject matter;
FIG. 1C illustrates a side view of an injection cartridge tip according to an embodiment of the presently disclosed subject matter;
FIG. 2A illustrates a perspective view of a device for delivery of one or more therapeutic agents to the subretinal space according to an embodiment of the presently disclosed subject matter;
FIG. 2B illustrates a top-down view of a device for delivery of one or more therapeutic agents to the subretinal space according to an embodiment of the presently disclosed subject matter;
FIG. 2C illustrates an enlarged view of a distal end of the device of FIG. 2B for delivery of one or more therapeutic agents to the subretinal space according to an embodiment of the presently disclosed subject matter;
FIG. 2D illustrates a front view of a distal end of the device of FIG. 2B for delivery of one or more therapeutic agents to the subretinal space according to an embodiment of the presently disclosed subject matter;
FIG. 2E illustrates a side view of a device for delivery of one or more therapeutic agents to the subretinal space according to an embodiment of the presently disclosed subject matter;
FIG. 2F illustrates an enlarged view of a distal end of the device of FIG. 2E for delivery of one or more therapeutic agents to the subretinal space according to an embodiment of the presently disclosed subject matter;
FIG. 3A illustrates a perspective view of a device for delivery of one or more therapeutic agents to the subretinal space according to an embodiment of the presently disclosed subject matter;
FIG. 3B illustrates an enlarged view of a distal end of the device of FIG. 3A for delivery of one or more therapeutic agents to the subretinal space according to an embodiment of the presently disclosed subject matter;
FIG. 3C illustrates an enlarged view of a distal end of the device of FIG. 3B for delivery of one or more therapeutic agents to the subretinal space according to an embodiment of the presently disclosed subject matter;
FIGS. 4A and 4B illustrate views a device for delivery of one or more therapeutic agents to the subretinal space according to an embodiment of the presently disclosed subject matter;
FIG. 5A is photograph of a representative embodiment of the presently disclosed CP-SSOCT distal-sensor guided injection device;
FIG. 5B is a photograph of a representative experimental setup of the presently disclosed CP-SSOCT distal-sensor guided injection device with a bovine eye;

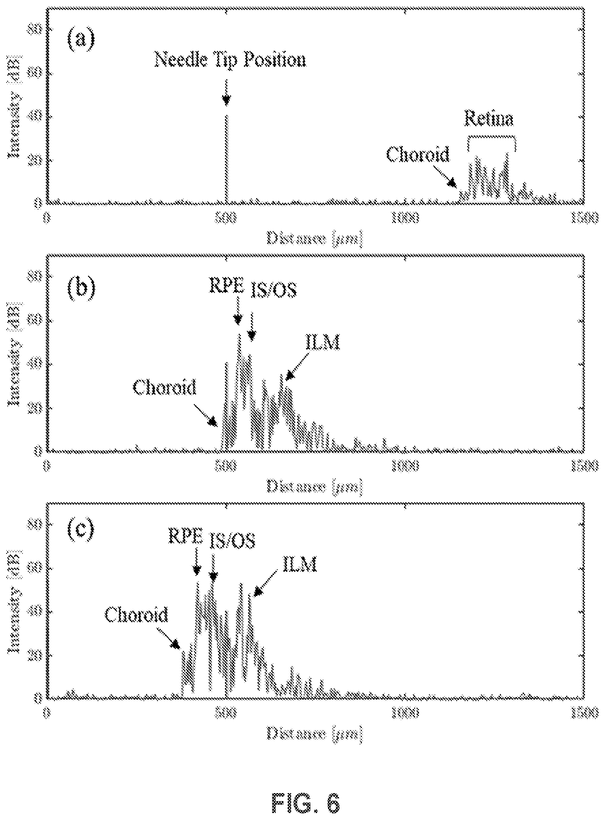

FIG. 6A is an A-Scan image of a bovine retina as the needle advances toward it at approximately 700 microns from the choroid;
FIG. 6B is an A-Scan image of a bovine retina as the needle touches the choroid; and
FIG. 6C is an A-Scan image of a bovine retina as the needle is inside the retina.

### DETAILED DESCRIPTION

The presently disclosed subject matter now will be described more fully hereinafter with reference to the accompanying Figures, in which some, but not all embodiments of the inventions are shown. Like numbers refer to like elements throughout. The presently disclosed subject matter may be embodied in many different forms and should not be construed as limited to the embodiments set forth herein; rather, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions and the associated Figures.

### I. EXTERNAL AND INTERNAL INTRAOPERATIVE OPTICAL COHERENCE TOMOGRAPHY IMAGING FOR SUBRETINAL MATERIAL DELIVERY

The subretinal space is a target location for the delivery of material, including therapeutic agents, such as genes, cells, such as stem cells, biologics, and small molecule therapeutics, into the eye. As used herein, the term "subretinal space" refers to the space between retinal pigment epithelium (RPE) cells and photoreceptors in the posterior segment of the eye. In the subretinal space, injected material, e.g., one or more therapeutic agents, comes into direct contact with the plasma membrane of the photoreceptor and RPE cells and subretinal blebs. The subretinal space is an excellent site for drug delivery, especially in patients with vision-threatening disorders attributable to mutations in photoreceptor and/or RPE genes and hereditary degenerative retinal diseases. See Peng et al., 2017.

The presently disclosed subject matter, in part, addresses the challenge of accessing the subretinal space directly from the outside of the eye through opaque structures that otherwise prevent visualization of the access route. More particularly, the presently disclosed subject matter provides a common-path swept source optical coherence tomography (CP-SSOCT) fiber probe sensor together with a precise translation stage and a graphical user interface that can guide the injection by providing the layer-by-layer information of the retina with micrometer accuracy without the need for a surgical microscope and intraoperative OCT.

The presently disclosed methods and devices allow a surgeon to access the subretinal space via an external approach (i.e., transsclerally through the white coat of the eye without internal eye surgery) with great precision using OCT. The presently disclosed device also allows the injection or placement of materials including, but not limited to fluids, gels, solids, cells, and other materials, into the subretinal space. The presently disclosed device allows a surgeon to access the subretinal space to create a subretinal "bleb," or small blister-like elevation, to facilitate the subsequent steps of retinal implantation surgery. The device allows the confirmation that the bleb has been created and the materials have been deposited in the correct location. The presently disclosed device is distinct from conventional ocular intraoperative OCT, which is designed for use through the pupil and mainly during internal eye surgery. In contrast, the presently disclosed device facilitates creation of an access route to the subretinal space from outside the eye.

The presently disclosed device and methods enable subretinal gene therapy and stem cell transplantation for age-related macular degeneration (AMD), including wet macular degeneration and dry macular degeneration, and other hereditary retinal diseases including, but not limited to, Leber's congenital amaurosis type 2 (LCA2), choroideremia, achromatopsia, retinitis pigmentosa (RP), Stargardt disease (STGD), Usher syndrome, juvenile X-linked retinoschisis (XLRS), and diabetic retinopathy.

Generally, OCT provides micrometer-levels of precision and subsurface imaging capability, as well as fast imaging speeds that can overcome existing limitations of microinjection techniques known in the art. See Sharma et al., 2005. A parallel programming technique using a graphics-processing unit (GPU) has been adopted for use with OCT to reduce the processing time for real-time guidance. See Zhang et al., 2010. The OCT distal sensing approach can be distinguished from typical OCT "B-scan" imaging in that distal sensing systems measure a distance value from acquired A-scan data. See Zhang et al., 2009; Zhang et al., 2011; Huang et al., 2011; Huang et al., 2012; Song et al., 2012; Song et al., 2013; and Cheon et al., 2015. In such systems, motion sensitive interference patterns are sequentially measured at consistent time intervals and continuously converted to a series of A-scans. The system counts the pixel distance in the image domain and then converts the number to a physical distance, which can be used to guide the position of the needle.

OCT probes suitable for use with the presently disclosed subject matter can include a swept source OEM engine (AXSUN, Billerica MA USA, central wavelength λ0: 1060 nm, sweeping rate: 100 kHz, 3 dB axial resolution: 8 µm, scan range: 3.7 mm in air), a balanced photodetector and a digitizer with a sampling rate of up to 500 MSPS with 12-bit resolution, a Camera Link DAQ Board, and a Camera Link frame grabber (PCIe-1433, National Instruments, Austin, TX USA). See Kang and Cheon, 2018.

Other OCT systems suitable for use with the presently disclosed devices and methods are disclosed in one or more of the following references:
U.S. Patent No. 10,045,882 for Surgical instrument and systems with integrated optical sensor, to Balicki et al., issued August 14, 2018;
U.S. Patent No. 10,039,530 for Interferometric force sensor for surgical instruments, to Taylor et al., issued August 7, 2018;
U.S. Patent No. 9,907,696 for Fiber optic distal sensor controlled micro-manipulation systems and methods, to Kang et al., issued March 6, 2018;
U.S. Patent No. 9,872,692 for Motion-compensated micro-forceps system and method, to Kang et al., issued January 23, 2018;
U.S. Patent No. 9,782,175 for Systems, methods and apparatuses for real-time anastomosis guidance and surgical evaluation using optical coherence tomography, to Kang et al., issued October 10, 2017;
U.S. Patent No. 9,506,741 for Optical coherence tomography systems and methods with magnitude and direction tracking of transverse motion, to Liu et al., issued November 29, 2016;
U.S. Patent No. 9,250,060 for Optical coherence tomography system having real-time artifact and saturation correction, to Kang et al., issued February 2, 2016;
U.S. Patent No. 9,243,887 for Lateral distortion corrected optical coherence tomography system, to Kang et al., issued January 26, 2016;
U.S. Patent No. 9,207,062 for Distortion corrected optical coherence tomography system, to Kang et al., issued December 8, 2015;
U.S. Patent No. 9,175,944 for Durable single mode fiber probe with optimized reference reflectivity, to Kang et al., issued November 3, 2015;
U.S. Patent No. 9,115,974 for Motion-compensated optical coherence tomography system, to Kang et al., issued August 25, 2015;
U.S. Patent No. 9,057,594 for Sapphire lens-based optical fiber probe for optical coherence tomography, to Kang et al., issued June 16, 2015;
U.S. Patent No. 8,921,767 for Automatic calibration of Fourier-domain optical coherence tomography systems, to Kang et al., issued December 30, 2014;
U.S. Patent Application Publication No. 20150209527 for Fiber optic distal sensor controlled drug injector, to Kang et al, published July 30, 2015; and
U.S. Patent Application Publication No. 20140039261 for Optical coherence tomography system and method for real-time surgical guidance, to Kang et al., published February 6, 2014.

In some embodiments, the presently disclosed subject matter provides a common-path swept source optical coherence tomography (CP-SSOCT) distal sensor-guided injection device comprising:
an optical fiber comprising a distal end, wherein the distal end of the optical fiber is configured to be proximate to or in contact with a selected portion of tissue, and wherein the optical fiber is arranged to direct light to the selected portion of tissue and to detect light reflected from the selected portion of tissue to provide information regarding a relative distance of the distal end of the optical fiber to the selected portion of tissue;
an injection needle configured to deliver one or more therapeutic agents to the selected portion of the tissue, wherein the injection needle is operably coupled and substantially parallel to the optical fiber such that the relative distance of the distal end of the optical fiber to the selected portion of tissue as determined by a CP-SSOCT system provides a position of a tip of the injection needle relative to the selected portion of tissue, wherein the tip comprises a distal end configured for insertion into the selected portion of tissue;
a translational stage configured to axially position the optical fiber and injection needle at a relative distance to the selected portion of tissue, wherein the optical fiber and injection needle are operably coupled to the translational stage; and
an articulated arm, wherein the translational stage is operably coupled to the articulated arm.

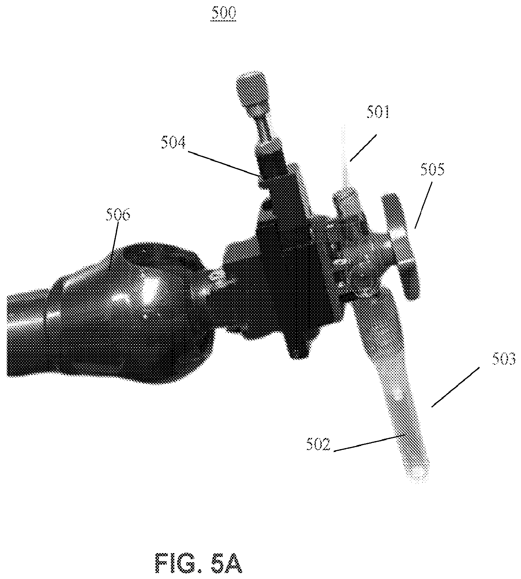

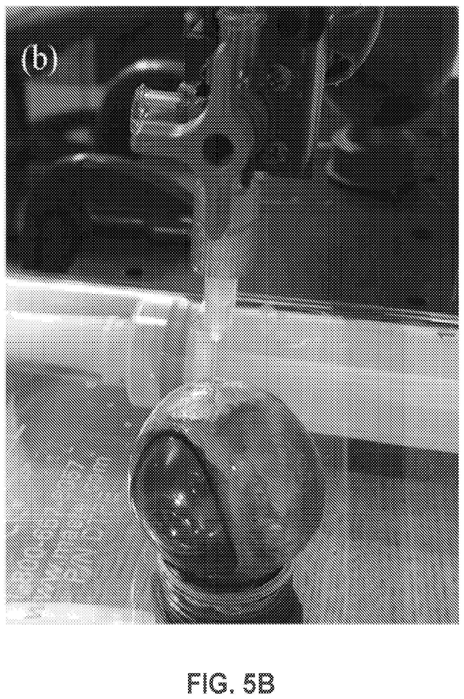

Referring now to FIG. 5 is a photograph of the presently disclosed system 500. System **500** includes OCT fiber **501** and needle **502,** which together comprise fiber/needle assembly **503.** System **500** further comprises translational stage **504** and a means for securing the fiber/needle assembly to translational stage **504.** In some embodiments, the means for securing the fiber/needle assembly **503** to translational stage **504** is a 3-way stopcock **505.** In some embodiments, translational stage **504** can be operably coupled to an articulated arm **506.**

In some embodiments, the device is adapted to be at least one of held by a surgeon for performing manual surgery or to be attached to a robotic system for at least one of robotic or robot-assisted surgery. In particular embodiments, the articulated arm is operably coupled to a robotic system. In more particular embodiments, the robotic system comprises a hand-held robotic system. In even yet more particular embodiments, the hand-held robotic system comprises a steady-hand robotic system.

In some embodiments, OCT fiber **501** further includes a high index epoxy lens on the distal end thereof (not shown). In some embodiments, needle **502** comprises a 30-gauge needle. One of ordinary skill in the art would recognize that needle **502** could be a 26- to 34-gauge needle, including 26-, 27-, 28-, 29-, 30-, 31-, 32-, 33-, and 34-gauge needle. In particular embodiments, needle **502** is a 26- to 30-gauge needle, including 26-, 27-, 28-, 29-, and 30-gauge needle. In yet more particular embodiments, needle **502** is a 29- or 30-gauge needle. In some embodiments, translational stage **504** has an accuracy ranging from about 25 micrometers to about 500 micrometers per revolution, including 25 µm, 50 µm, 100 µm, 150 µm, 200 µm, 250 µm, 300 µm, 350 µm, 400 µm, 450 µm, and 500 µm. In some embodiments, translational stage **504** has an accuracy of 250 µm per revolution. In some embodiments, translational stage **504** has a positional sensitivity of about 1 µm. Translational stage **504** can be manually operated or motor driven.

In some embodiments, fiber/needle assembly **503** can include one or more embodiments disclosed in U.S. provisional patent application no. 62/714,280 to Singh et al., for RETINAL IMPLANT DEVICE, filed August 3, 2018. More particularly, the presently disclosed device can include a retinal implantation or delivery device configured to move through a subretinal space of an eye and dispense materials therein. Such a device can be formed from a flexible material that allows the device to curve along the subretinal space.

More particularly, and as provided in U.S. provisional patent application no. 62/714,280 to Singh et al., the device can include two stacked layers comprising a pair of thin, elongate strips of flexible material, which are surrounded by a flexible outer surface comprising a thin layer of elastic material thereby forming a flexible, expandable tube. The device further comprises an elongate lumen extending between the pair of thin, elongate strips of flexible material. The elongate lumen is configured for dispensing material through the device and into the subretinal space.

In some embodiments, the pair of thin, elongate strips are formed from polyimide, although one of ordinary skill in the art would recognize that other materials would be suitable for use with the presently disclosed device. In some embodiments, the thin layer of flexible material surrounding the pair of thin, elongate strips is formed from latex, although one of ordinary skill in the art would recognize that other materials would be suitable for use with the presently disclosed device. A geometry of the device is configured such that the device is flexible in one bending direction, but rigid or less flexible in another, e.g., opposite, direction. Thus, the device is configured to be flexible and bendable in one direction such that is can conform to the natural curvature of the eye as the device is advanced to the subretinal space. The device also can include a flexible cannula to safely navigate the curved propagation tunnel Once the device is in place, the flexible outer layer is configured to protect the delicate tissue of the retina, retinal pigment epithelium, and choroid A microfluidic dispensing system also can be used in lieu of a plunger system for delivery of material through the device.

A distal end of the pair of thin, elongate strips of flexible material can have a rounded shape. The device can further include fixation holes along each edge of the strips of flexible material that can be used to secure the device to the eye during the insertion procedure.

Because the sclera is opaque, the device can further include an OCT sensor for visualization of an entry point of the device. such that a user can determine a depth of penetration of the device upon entry, which can continue along the delivery path to the target point, e.g., the subretinal space.

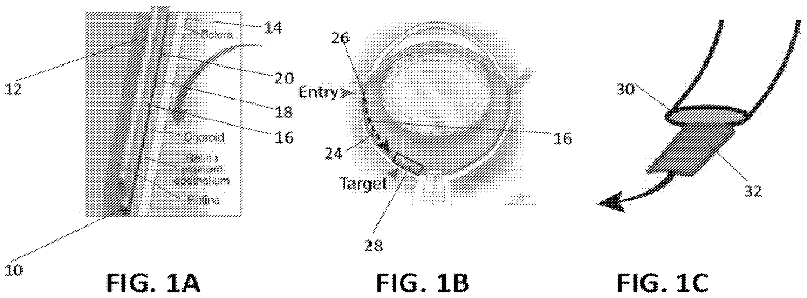

Referring now to FIG. 1A, FIG. 1B, and FIG. 1C, is provided a partially sectional view of a plane of delivery representative of the presently disclosed methods. As illustrated in FIG. 1A, a delivery path 10 extends between retina 12 and sclera 14 in subretinal space 16. Subretinal space 16 can be accessed through either the scleral side or the subretinal side

Referring now to FIG. 1B, is a partially sectional view of an entry point and a target point according to an embodiment of the presently disclosed subject matter. In such embodiments, the presently disclosed device travels along a curved trajectory **24** in subretinal space **16** between entry point **26** and target point **28.** Curved trajectory **24** in subretinal space **16** avoids trauma to the retina **12,** retinal pigment epithelium **20,** and choroid **18** (as shown in FIG. 1A).

Referring now to FIG. 1C, is a side view of an injection cartridge tip **30,** according to an embodiment of the presently disclosed subject matter. In certain embodiments, injection cartridge tip **30** is advanced along the curved trajectory **24** to target point **28** as described in FIG. 1A and FIG. 1B. Injection cartridge tip **30** thereby delivers material **32** to target point **28** in a manner that is minimally traumatic to target point **28** and/or the material **32** to be delivered. The device and injection cartridge tip **30** can be configured to inject a planar sheet of material **32.** In some embodiments, for example, for the delivery of stem cells, such a configuration allows for the delivery of the stem cells in the correct apical-basal orientation. One design feature of the presently disclosed device adapted to achieve this result is the use of an elliptical cross section of injection cartridge tip **30**

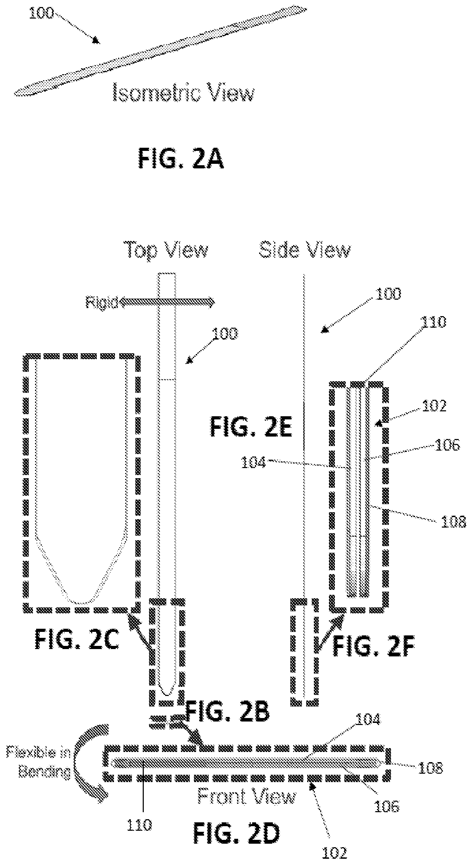

Referring now to FIGS. 2A-2F are representative views of the presently disclosed device **100** for delivery of materials to the subretinal space. FIG. 2A illustrates a perspective or isometric view of device **100.** FIG. 2B illustrates a top-down view of an embodiment of device **100.** FIG. 2C illustrates an enlarged view of a distal end of device **100** of FIG. 2B. FIG. 2D illustrates a front view of a distal end of device 100 of FIG. 2B. FIG. 2E illustrates a side view of device **100.** FIG. 2F illustrates an enlarged view of a distal end of device **100** of FIG. 2E.

Referring once again to FIGS. 2A-2F, device **100** includes tube **102.** Tube **102** includes two flat strips **104, 106** jacketed by a thin layer of elastic material **108,** which, in some embodiments, is formed from latex. The two flat strips **104, 106** are formed from a flexible material, e.g., polyimide, that allows device **100** to curve along the subretinal space. A thin, flat lumen **110** is defined between the two flat strips **104, 106** and allows for dispensing material or cells through device **100.** While a thin flat lumen is described as an example herein, this is merely one embodiment, and any lumen shape known to or conceivable by one of skill in the art could also be used. The geometry of device **100** allows it to be flexible in one bending direction, but more rigid in the other direction. The multilayer structure and elastic outer jacket **108** of device **100** allows materials to be passed through lumen **110.**

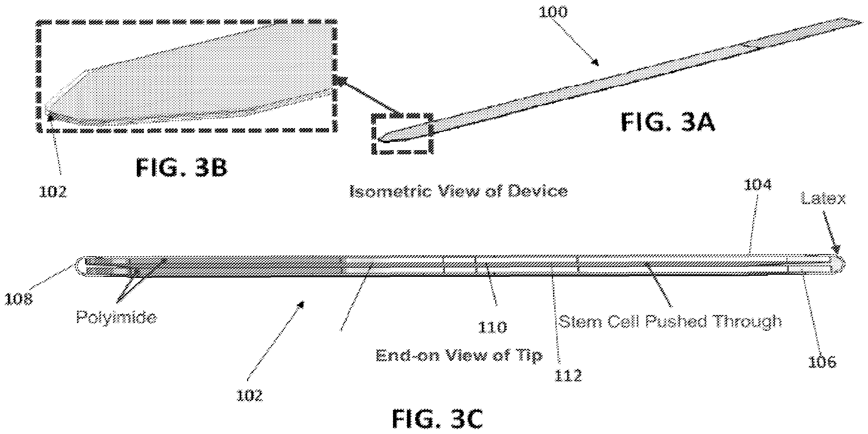

FIGS. 3A-3C illustrate further views of device **100.** FIG. 3A illustrates a perspective view of device **100.** FIG. 3B illustrates an enlarged view of a distal end of device **100** of FIG. 3A. FIG. 3C illustrates an enlarged end-on view of a distal end of device **100.** As illustrated in FIGS. 3A-3C, device **100** includes tube **102.** Tube **102** includes two flat strips **104, 106** jacketed by a thin layer of elastic material **108.** The two flat strips **104, 106** are formed from a flexible material that allows device **100** to curve along the subretinal space. As also shown in FIG. 3C, a thin, flat lumen **110** is defined between the two flat strips **104, 106** and allows for dispensing of material through device **100.** As illustrated in FIG. 3C, material **112** can be pushed through the space defined by the two flat strips **104, 106.** Tube **102,** as configured, is intended to slide into the sub-retinal space to allow delivery of therapeutic agents for treating various retinal eye diseases or disorders.

Accordingly, in some embodiments, an incision is made in the sclera and choroid to access the subretinal space. Tube **102** is then inserted into the space between the choroid and retina. Because of its flexibility, tube **102** can be manually inserted and will conform to the curvature of the eye. After tube **102** is in place, the thin layer of elastic material **108,** in some embodiments, latex, protects the delicate tissues of the retina and choroid, while, in some embodiments, the polyimide forming the two flat strips **104, 106** allows the easy passage of objects between the two polyimide layers. The leading edge of the device is rounded to help separate the retina and choroid with minimal trauma.

The system can further include lubricating material between the components to facilitate smooth passage and prevent unwanted adhesion of the material to be delivered. The system also can include a hub for facilitating the injection of material to the retina. The hub can include a reservoir for the material, such as a syringe or other reservoir known to or conceivable to one of skill in the art. In such embodiments, the reservoir is in fluid communication with the injection needle, wherein the reservoir is configured to move fluid through the injection needle to the selected portion of tissue. In particular embodiments, the reservoir comprises a syringe or a microfluidic device.

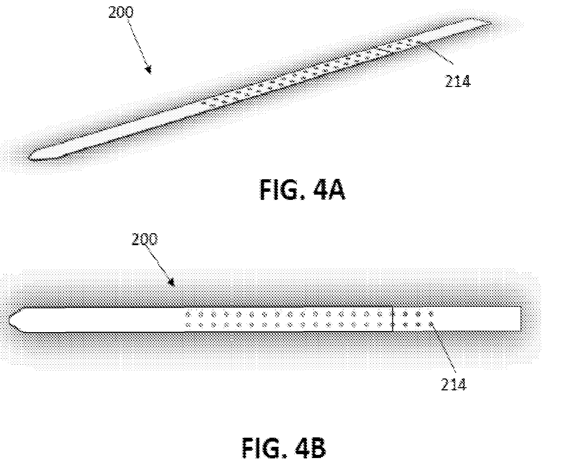

Referring now to FIGS. 4A and 4B are representative views of the presently disclosed device **200** for delivery of material to the subretinal space. FIG. 4A illustrates a perspective view of device **200** and FIG. 4B illustrates a top down view of device **200.** As illustrated in FIGS. 4A and 4B, device **200** includes fixation holes **214** along each edge of the strips of flexible material that are used to secure the device to the eye during the insertion procedure.

In further embodiments, the presently disclosed device further comprises a graphical user interface. In particular embodiments, graphical user interface is configured to provide a value of the relative distance of the distal end of the optical fiber to the selected portion of tissue or a relative position of the tip of the injection needle to the selected portion of tissue.

In some embodiments, a graphical user interface (GUI) or graphics processing unit (GPU) can be used to reduce the processing time for real-time guidance of the position of the needle relative to the target. See Zhang and Kang, 2010. More particularly, the GUI or GPU can process the OCT spectral data to facilitate the distal sensing. Details of representative signal processing procedures are provided in Cheon et al., 2015. In some embodiments, the spectral data are transformed to A-scan data. For example, if the sweeping rate is 100 kHz, the OCT engine returns 128 buffered spectral data every 1.28 ms. These oversampled data are useful to increase the signal-to-noise ratio because the temporally and spatially averaged data within a restricted window based on target size and speed effectively remove speckle noise, which is the one of the main noise factors in A-scan. The increased processing time due to the oversampled data, however, has serious adverse effects on intraoperative guidance capabilities. Accordingly, parallel processing using a GPU significantly reduces the processing time.

For example, consecutive spectra transmitted from the frame grabber to the GPU in the workstation can be passed to a high-pass filter to remove the high DC component in the spectral data. Then, low-pass filtering can be consecutively applied to remove the high-frequency noise components. Next, zero padding, FFT, averaging, and background subtraction can be conducted on the A-scan data. These steps produce an A-scan with a certain number of data points that cover a certain axial distance from the distal end of the fiber probe. The processed A-scan is passed to the next step that detects the position of the tissue surface.

The A-scan of the tissue has a multilayered structure with a complex signature. Thus, surface detection in combination with a shifted cross-correlation method can be used to determine the absolute position of the surface and to determine the distance variation (relative position) between temporally adjacent data. This method is advantageous in that it uses the features of the A-scan that reflect deep-tissue anatomical structure, and detect distance variance robustly, where each A-scan point is vulnerable to noise. Finally, the calculated position value is transmitted to the third step for motion control.

Instead of using the position value directly to control the axial position, the output of a Kalman predictor is used to make up for the computational and communication time delay between the spectrum measurement and the actuator operation. The axial motion guidance can be controlled based on the distance between needle tip and sample surface. See Kang and Cheon, 2018.

The presently disclosed device and methods can be carried out using a computer, non-transitory computer readable medium, or alternately a computing device or non-transitory computer readable medium incorporated into the system. Indeed, any suitable method of calculation known to or conceivable by one of skill in the art could be used. A nontransitory computer readable medium is understood to mean any article of manufacture that can be read by a computer. Such non-transitory computer readable media includes, but is not limited to, magnetic media, such as a floppy disk, flexible disk, hard disk, reel-to-reel tape, cartridge tape, cassette tape or cards, optical media such as CD-ROM, writable compact disc, magnetooptical media in disc, tape or card form, and paper media, such as punched cards and paper tape. The computing device can be a special computer designed specifically for this purpose. The computing device can be unique to the present invention and designed specifically to carry out the method of the present invention.

Imagers, such as the optical coherence tomography sensor described herein, generally have a console, which is a proprietary master control center of the sensor designed specifically to carry out the operations of the imaging and receive the imaging data created by the sensor. Typically, this console is made up of a specialized computer, custom keyboard, and multiple monitors. There can be two different types of control consoles, one used by the OCT operator and the other used by the physician. The operator's console controls such variables as the thickness of the image, the amount of tube current/voltage, mechanical movement of the patient table and other technique factors. The physician's viewing console allows viewing of the images without interfering with the normal OCT imaging operation. This console is capable of rudimentary image analysis. The operating console computer is a nongeneric computer specifically designed for bilateral (input output) communication with the imager. It is not a standard business or personal computer that can be purchased at a local store. Additionally. this console computer carries out communications with the imager through the execution of proprietary custom-built software that is designed and written for the computer hardware to specifically operate the imager hardware.

As used herein, the term "treating" can include reversing, alleviating, inhibiting the progression of, preventing or reducing the likelihood of the disease, disorder, or condition to which such term applies, or one or more symptoms or manifestations of such disease, disorder or condition. Preventing refers to causing a disease, disorder, condition, or symptom or manifestation of such, or worsening of the severity of such, not to occur. Accordingly, the presently disclosed compounds can be administered prophylactically to prevent or reduce the incidence or recurrence of the disease, disorder, or condition.

The "subject" treated by the presently disclosed methods in their many embodiments is desirably a human subject, although it is to be understood that the methods described herein are effective with respect to all vertebrate species, which are intended to be included in the term "subject." Accordingly, a "subject" can include a human subject for medical purposes, such as for the treatment of an existing condition or disease or the prophylactic treatment for preventing the onset of a condition or disease, or an animal subject for medical, veterinary purposes, or developmental purposes. Suitable animal subjects include mammals including, but not limited to, primates, e.g., humans, monkeys, apes, and the like; bovines, e.g., cattle, oxen, and the like; ovines, e.g., sheep and the like; caprines, e.g., goats and the like; porcines, e.g., pigs, hogs, and the like; equines, e.g., horses, donkeys, zebras, and the like; felines, including wild and domestic cats; canines, including dogs; lagomorphs, including rabbits, hares, and the like; and rodents, including mice, rats, and the like. An animal may be a transgenic animal. **In** some embodiments, the subject is a human including, but not limited to, fetal, neonatal, infant, juvenile, and adult subjects. Further, a "subject" can include a patient afflicted with or suspected of being afflicted with a condition or disease. Thus, the terms "subject" and "patient" are used interchangeably herein. The term "subject" also refers to an organism, tissue, cell, or collection of cells from a subject.

Following long-standing patent law convention, the terms "a," "an," and "the" refer to "one or more" when used in this application, including the claims. Thus, for example, reference to "a subject" includes a plurality of subjects, unless the context clearly is to the contrary (e.g., a plurality of subjects), and so forth.

Throughout this specification and the claims, the terms "comprise," "comprises," and "comprising" are used in a non-exclusive sense, except where the context requires otherwise. Likewise, the term "include" and its grammatical variants are intended to be non-limiting, such that recitation of items in a list is not to the exclusion of other like items that can be substituted or added to the listed items.

For the purposes of this specification and appended claims, unless otherwise indicated, all numbers expressing amounts, sizes, dimensions, proportions, shapes, formulations, parameters, percentages, quantities, characteristics, and other numerical values used in the specification and claims, are to be understood as being modified in all instances by the term "about" even though the term "about" may not expressly appear with the value, amount or range. Accordingly, unless indicated to the contrary, the numerical parameters set forth in the following specification and attached claims are not and need not be exact, but may be approximate and/or larger or smaller as desired, reflecting tolerances, conversion factors, rounding off, measurement error and the **like, and** other factors known to those of skill in the art depending on the desired properties sought to be obtained by the presently disclosed subject matter. For example, the term "about," when referring to a value can be meant to encompass variations of, in some embodiments, ± 100% in some embodiments ± 50%, in some embodiments ± 20%, in some embodiments ± 10%, in some embodiments ± 5%, in some embodiments ±1%, in some embodiments ± 0.5%, and in some embodiments ± 0.1% from the specified amount, as such variations are appropriate to perform the disclosed methods or employ the disclosed compositions.

Further, the term "about" when used in connection with one or more numbers or numerical ranges, should be understood to refer to all such numbers, including all numbers in a range and modifies that range by extending the boundaries above and below the numerical values set forth. The recitation of numerical ranges by endpoints includes all numbers, e.g., whole integers, including fractions thereof, subsumed within that range (for example, the recitation of 1 to 5 includes 1, 2, 3, 4, and 5, as well as fractions thereof, e.g., 1.5, 2.25, 3.75, 4.1, and the like) and any range within that range.

### EXAMPLES

The following Examples have been included to provide guidance to one of ordinary skill in the art for practicing representative embodiments of the presently disclosed subject matter. **In** light of the present disclosure and the general level of skill in the art, those of skill can appreciate that the following Examples are intended to be exemplary only and that numerous changes, modifications, and alterations can be employed without departing from the scope of the presently disclosed subject matter. The synthetic descriptions and specific examples that follow are only intended for the purposes of illustration and are not to be construed as limiting in any manner to make compounds of the disclosure by other methods.

### EXAMPLE 1

### Optical Coherence Tomography Distal-Sensor Guided Manual Injection Device for Transscleral Subretinal Injection

*1.1 Methods.* The CP-SSOCT sensor with a high index epoxy lens on the end surface was placed inside a 30-gauge needle. A three-way stopcock was used to allow for a fiber going through the tube and for a syringe to be connected to perform the subretinal injection. The needle and the stopcock were mounted on a precise translation stage with accuracy of 250 microns per revolution. The test was performed on bovine eyes with the device fixed on the articulated flexible arm. Part of the sclera had been removed and the needle was moved towards to eye by the translation stage. The A-scan images of bovine retina were obtained at each step of insertion to determine whether the layer information of retina can be acquired.

*1.2 Results.* The experiment was set up with bovine eyes and the needle positioned vertically with respect to the eye. A-scan images of bovine retina were acquired as the needle was moving toward it. Before the insertion, the retinal signal was clear. When the needle made contact with the choroid, the layers of the retina could be identified. Using the peak and envelop information, the retinal pigment epithelium (RPE), junction of photoreceptor inner and outer segments (IS/OS), and the inner limiting membrane (ILM) could be labeled on the A-scan image. As the needle was inside the sclera, the layers remained clearly identifiable. The retinal thickness as measured by the fiber sensor was around 300 µm, which is comparable to that measured by a reference-based OCT imaging system.

*1.3 Summary.* The presently disclosed results demonstrate that a CP-SSOCT fiber sensor can guide needle insertion into the subretinal space for transscleral injection. The device is compact and flexible enough to be mounted within the typical vitreoretinal surgery setup in an operating room to achieve safe and precise access to the subretinal space for therapeutic procedures.

### REFERENCES

All publications, patent applications, patents, and other references mentioned in the specification are indicative of the level of those skilled in the art to which the presently disclosed subject matter pertains.

Cheon, G. W., Huang, Y., Cha, J., Gehlbach, P.L., and Kang, J.U., Accurate Real-Time Depth Control for CP-SSOCT Distal Sensor Based Handheld Microsurgery Tools, Biomedical Optics Express 2015, 6(5):1942-1953.

Huang, Y.; Zhang, K.; Lin, C.; Kang, J.U. Motion compensated fiber-optic confocal microscope based on a common-path optical coherence tomography distance sensor. Opt. Eng. 2011, 50, 083201.

Huang, Y.; Liu, X.; Song, C.; Kang, J.U. Motion-compensated hand-held common-path Fourier-domain optical coherence tomography probe for image-guided intervention. Biomed. Opt. Express 2012, 3, 3105-3118.

Kang, J.U., Han, J.-H., and Zhang, K., Common-Path Optical Coherence for Biomedical Imagining and Sensing, J. Opt. Soc. Korea, 2010, 14(1), 1-13.

Kang, J.U. and Cheon, G. W., Demonstration of subretinal injection suing common-path swept source OCT guides microinjector, Appl. Sci. 2018, 8, 1287.

Peng, Y., Tang L., Zhou, Y. Subretinal Injection: A Review on the Novel Route of Therapeutic Delivery for Vitreoretinal Diseases, Ophthalmic Res 2017;58:217-226.

Sharma, U.; Fried, N.M.; Kang, J.U. All-Fiber Common-Path Optical Coherence Tomography: Sensitivity Optimization and System Analysis. IEEE J. Sel. Top. Quantum Electron. 2005, 11, 799-805.

Song, C.; Gehlbach, P.L.; Kang, J.U. Active tremor cancellation by a "smart" handheld vitreoretinal microsurgical tool using swept source optical coherence tomography. Opt. Express 2012, 20, 23414-23421.

Song, C.; Park, D.Y.; Gehlbach, P.L.; Park, S.J.; Kang, J.U. Fiber-optic OCT sensor guided "SMART" micro-forceps for microsurgery. Biomed. Opt. Express 2013, 4, 1045-1050.

Zhang, K.; Wang, W.; Han, J.; Kang, J.U. A surface topology and motion compensation system for microsurgery guidance and intervention based on common-path optical coherence tomography. IEEE Trans. Biomed. Eng. 2009, 56, 2318-2321.

Zhang, K.; Kang, J.U. Graphics processing unit accelerated non-uniform fast Fourier transform for ultrahigh-speed, real-time Fourier-domain OCT. Opt. Express 2010, 18, 23472-23487.

Zhang, K.; Kang, J.U. Common-path low-coherence interferometry fiber-optic sensor guided microincision. J. Biomed. Opt. 2011, 16, 095003.

International PCT Patent Application Publication No. WO2015161101 for Fiber Optic Distal Sensor Controlled Micro-Manipulation Systems and Methods, to Kang and Gehlbach, published October 22, 2015.

U.S. Patent No. 10,045,882 for Optical Coherence Tomography System, to Balicki, issued August 14, 2018.

Although the foregoing subject matter has been described in some detail by way of illustration and example for purposes of clarity of understanding, it will be understood by those skilled in the art that certain changes and modifications can be practiced within the scope of the appended claims.

## Claims

1. A common-path swept source optical coherence tomography (*CP-SSOCT*) distal sensor-guided injection device (500) comprising:
an optical fiber (501) comprising a distal end, wherein the distal end of the optical fiber is configured to be proximate to or in contact with a selected portion of tissue, and wherein the optical fiber is arranged to direct light to the selected portion of tissue and to detect light reflected from the selected portion of tissue to provide information regarding a relative distance of the distal end of the optical fiber to the selected portion of tissue;
an injection needle (502) configured to deliver one or more therapeutic agents to the selected portion of the tissue, wherein the injection needle is operably coupled and substantially parallel to the optical fiber such that the relative distance of the distal end of the optical fiber to the selected portion of tissue as determined by a *CP-SSOCT* system provides a position of a tip of the injection needle relative to the selected portion of tissue, wherein the tip comprises a distal end configured for insertion into the selected portion of tissue;
a translational stage (504) configured to axially position the optical fiber and injection needle at a relative distance to the selected portion of tissue, wherein the optical fiber and injection needle are operably coupled to the translational stage; and
an articulated arm (506), wherein the translational stage is operably coupled to the articulated arm;
and **characterized in that** the needle tip further comprises an injection needle cartridge tip (30) comprising:
a pair of thin, elongate strips of flexible material (104, 106);
a thin layer of elastic material (108) surrounding the pair of thin, elongate strips of flexible material;
wherein the pair of thin, elongate strips of flexible material and thin layer of elastic material surrounding the pair of thin, elongate strips of flexible material form a flexible, expandable tube (102), wherein an elongate lumen (110) extends between the pair of thin, elongate strips of flexible material and wherein the elongate lumen is configured to dispense one or more therapeutic agents to a target location of the selected portion of tissue.

2. The device of claim 1, wherein the device is configured to move through a subretinal space (16) of an eye.

3. The device of claim 2, wherein the device is configured to access a subretinal space of an eye transsclerally.

4. The device of claim 1, wherein the device is configured to deliver one or more therapeutic agents to a subretinal space of an eye.

5. The device of claim 4, wherein the one or more therapeutic agents is selected from a gene, a cell, a biologic, and a small molecule therapeutic agent.

6. The device of claim 1, wherein the device is adapted to be at least one of held by a surgeon for performing manual surgery or to be attached to a robotic system for at least one of robotic or robot-assisted surgery.

7. The device of claim 1, wherein the articulated arm is operably coupled to a robotic system.

8. The device of claim 7, wherein the robotic system comprises a hand-held robotic system.

9. The device of claim 8, wherein the hand-held robotic system comprises a steady-hand robotic system.

10. The device of claim 1, further comprising a graphical user interface.

11. The device of claim 10, wherein the graphical user interface is configured to provide a value of the relative distance of the distal end of the optical fiber to the selected portion of tissue or a relative position of the tip of the injection needle to the selected portion of tissue.

12. The device of claim 1, further comprising a reservoir in fluid communication with the injection needle, wherein the reservoir is configured to move fluid through the injection needle to the selected portion of tissue.

13. The device of claim 12, wherein the reservoir comprises a syringe or a microfluidic device.

## Patentansprüche

1. Eine mittels eines distalen Sensors der optischen Common-Path-Swept-Source-Kohärenztomographie (*CP-SSOCT,* optische Kohärenztomographie mit gemeinsamem Strahlenweg und durchstimmbarer Strahlungsquelle) geführte Injektionsvorrichtung (500), die Folgendes beinhaltet:
eine Lichtleitfaser (501), die ein distales Ende beinhaltet, wobei das distale Ende der Lichtleitfaser konfiguriert ist, um nahe oder in Kontakt mit einem ausgewählten Gewebeabschnitt zu sein, und wobei die Lichtleitfaser eingerichtet ist, um Licht auf den ausgewählten Gewebeabschnitt zu lenken und von dem ausgewählten Gewebeabschnitt reflektiertes Licht zu detektieren, um Informationen bezüglich einer relativen Distanz des distalen Endes der Lichtleitfaser zu dem ausgewählten Gewebeabschnitt bereitzustellen;
eine Injektionsnadel (502), die konfiguriert ist, um ein oder mehrere Therapeutika an den ausgewählten Abschnitt des Gewebes zu liefern, wobei die Injektionsnadel betriebsfähig mit der Lichtleitfaser gekoppelt und dazu im Wesentlichen parallel ist,
sodass die relative Distanz des distalen Endes der Lichtleitfaser zu dem ausgewählten Gewebeabschnitt, wie durch ein *CP-SSOCT*-System bestimmt, eine Position einer Spitze der Injektionsnadel relativ zu dem ausgewählten Gewebeabschnitt bereitstellt, wobei die Spitze ein distales Ende beinhaltet, das zur Einführung in den ausgewählten Gewebeabschnitt konfiguriert ist;
einen Verschiebetisch (504), der konfiguriert ist, um die Lichtleitfaser und die Injektionsnadel in einer relativen Distanz zu dem ausgewählten Gewebeabschnitt axial zu positionieren, wobei die Lichtleitfaser und die Injektionsnadel betriebsfähig mit dem Verschiebetisch gekoppelt sind; und
einen Gelenkarm (506), wobei der Verschiebetisch betriebsfähig mit dem Gelenkarm gekoppelt ist;
und **dadurch gekennzeichnet, dass** die Nadelspitze ferner eine Injektionsnadeleinsatzspitze (30) beinhaltet, die Folgendes beinhaltet:
ein Paar dünne, längliche Streifen flexiblen Materials (104, 106);
eine dünne Schicht elastischen Materials (108), die das Paar dünne, längliche Streifen flexiblen Materials umgibt;
wobei das Paar dünne, längliche Streifen flexiblen Materials und die dünne Schicht elastischen Materials, die das Paar dünne, längliche Streifen flexiblen Materials umgibt, eine flexible, dehnbare Röhre (102) bilden, wobei sich zwischen dem Paar dünnen, länglichen Streifen flexiblen Materials ein längliches Lumen (110) erstreckt und wobei das längliche Lumen konfiguriert ist, um ein oder mehrere Therapeutika an eine Zielstelle des ausgewählten Gewebeabschnitts abzugeben.

2. Vorrichtung gemäß Anspruch 1, wobei die Vorrichtung konfiguriert ist, um sich durch einen subretinalen Raum (16) eines Auges zu bewegen.

3. Vorrichtung gemäß Anspruch 2, wobei die Vorrichtung konfiguriert ist, um transskleral Zugang zu einem subretinalen Raum eines Auges zu erlangen.

4. Vorrichtung gemäß Anspruch 1, wobei die Vorrichtung konfiguriert ist, um ein oder mehrere Therapeutika an einen subretinalen Raum eines Auges zu liefern.

5. Vorrichtung gemäß Anspruch 4, wobei das eine oder die mehreren Therapeutika aus einem Gen, einer Zelle, einem Biologikum und einem Kleinmolekül-Therapeutikum ausgewählt sind.

6. Vorrichtung gemäß Anspruch 1, wobei die Vorrichtung für mindestens eines von Folgendem angepasst ist: um von einem Chirurgen zum Durchführen manueller Chirurgie gehalten zu werden oder um an einem Robotiksystem für mindestens eines von Roboter- oder roboterassistierter Chirurgie angebracht zu sein.

7. Vorrichtung gemäß Anspruch 1, wobei der Gelenkarm betriebsfähig mit einem Robotiksystem gekoppelt ist.

8. Vorrichtung gemäß Anspruch 7, wobei das Robotiksystem ein handgeführtes Robotiksystem beinhaltet.

9. Vorrichtung gemäß Anspruch 8, wobei das handgeführte Robotiksystem ein Steady-Hand-Robotiksystem beinhaltet.

10. Vorrichtung gemäß Anspruch 1, die ferner eine graphische Benutzeroberfläche beinhaltet.

11. Vorrichtung gemäß Anspruch 10, wobei die graphische Benutzeroberfläche konfiguriert ist, um einen Wert der relativen Distanz des distalen Endes der Lichtleitfaser zu dem ausgewählten Gewebeabschnitt oder eine relative Position der Spitze der Injektionsnadel zu dem ausgewählten Gewebeabschnitt bereitzustellen.

12. Vorrichtung gemäß Anspruch 1, die ferner einen Behälter in Fluidkommunikation mit der Injektionsnadel beinhaltet, wobei der Behälter konfiguriert ist, um Fluid durch die Injektionsnadel zu dem ausgewählten Gewebeabschnitt zu bewegen.

13. Vorrichtung gemäß Anspruch 12, wobei der Behälter eine Spritze oder eine mikrofluidische Vorrichtung beinhaltet.

## Revendications

1. Un dispositif d'injection guidé par capteur distal (500) de tomographie par cohérence optique à source balayée à trajet commun (*CP-SSOCT*) comprenant :
une fibre optique (501) comprenant une extrémité distale, dans lequel l'extrémité distale de la fibre optique est configurée pour être proximale à ou en contact avec une portion sélectionnée de tissu, et dans lequel la fibre optique est disposée de manière à diriger de la lumière vers la portion sélectionnée de tissu et détecter de la lumière réfléchie depuis la portion sélectionnée de tissu afin de fournir des informations concernant une distance relative de l'extrémité distale de la fibre optique par rapport à la portion sélectionnée de tissu ;
une aiguille d'injection (502) configurée pour livrer un ou plusieurs agents thérapeutiques dans la portion sélectionnée du tissu, dans lequel l'aiguille d'injection est fonctionnellement couplée et substantiellement parallèle à la fibre optique de telle sorte que la distance relative de l'extrémité distale de la fibre optique par rapport à la portion sélectionnée de tissu tel que déterminé par un système de CP-SSOCT fournit une position d'une pointe de l'aiguille d'injection par rapport à la portion sélectionnée de tissu, dans lequel la pointe comprend une extrémité distale configurée pour être insérée jusque dans la portion sélectionnée de tissu ;
une platine translationnelle (504) configurée pour positionner axialement la fibre optique et l'aiguille d'injection à une distance relative par rapport à la portion sélectionnée de tissu, dans lequel la fibre optique et l'aiguille d'injection sont fonctionnellement couplées à la platine translationnelle ; et
un bras articulé (506), dans lequel la platine translationnelle est fonctionnellement couplée au bras articulé ;
et **caractérisé en ce que** la pointe d'aiguille comprend en sus une pointe à cartouche d'aiguille d'injection (30) comprenant :
une paire de bandes allongées fines de matériau flexible (104, 106) ;
une couche fine de matériau élastique (108) entourant la paire de bandes allongées fines de matériau flexible ;
dans lequel la paire de bandes allongées fines de matériau flexible et la couche fine de matériau élastique entourant la paire de bandes allongées fines de matériau flexible forment un tube dilatable flexible (102), dans lequel une lumière allongée (110) s'étend entre la paire de bandes allongées fines de matériau flexible et dans lequel la lumière allongée est configurée pour dispenser un ou plusieurs agents thérapeutiques dans un endroit cible de la portion sélectionnée de tissu.

2. Le dispositif de la revendication 1, le dispositif étant configuré pour se déplacer à travers un espace sous-rétinien (16) d'un œil.

3. Le dispositif de la revendication 2, le dispositif étant configuré pour accéder à un espace sous-rétinien d'un œil par voie transsclérale.

4. Le dispositif de la revendication 1, le dispositif étant configuré pour livrer un ou plusieurs agents thérapeutiques dans un espace sous-rétinien d'un œil.

5. Le dispositif de la revendication 4, dans lequel les uns ou plusieurs agents thérapeutiques sont sélectionnés parmi un gène, une cellule, un agent thérapeutique biologique, et un agent thérapeutique à petite molécule.

6. Le dispositif de la revendication 1, dans lequel le dispositif est conçu pour être au moins soit un dispositif tenu par un chirurgien pour réaliser une intervention chirurgicale manuelle, soit un dispositif destiné à être rattaché à un système robotique pour au moins soit une intervention chirurgicale robotique, soit une intervention chirurgicale assistée par robot.

7. Le dispositif de la revendication 1, dans lequel le bras articulé est fonctionnellement couplé à un système robotique.

8. Le dispositif de la revendication 7, dans lequel le système robotique comprend un système robotique à main.

9. Le dispositif de la revendication 8, dans lequel le système robotique à main comprend un système robotique à main sûre.

10. Le dispositif de la revendication 1, comprenant en sus une interface utilisateur graphique.

11. Le dispositif de la revendication 10, dans lequel l'interface utilisateur graphique est configurée pour fournir une valeur de la distance relative de l'extrémité distale de la fibre optique par rapport à la portion sélectionnée de tissu ou une position relative de la pointe de l'aiguille d'injection par rapport à la portion sélectionnée de tissu.

12. Le dispositif de la revendication 1, comprenant en sus un réservoir en communication fluidique avec l'aiguille d'injection, dans lequel le réservoir est configuré pour faire circuler du fluide à travers l'aiguille d'injection jusqu'à la portion sélectionnée de tissu.

13. Le dispositif de la revendication 12, dans lequel le réservoir comprend une seringue ou un dispositif microfluidique.
